Europäisches Patentamt

European Patent Office

Office européen des brevets

(19)

(11) **EP 1 524 508 A1**

(12) **EUROPEAN PATENT APPLICATION**

(43) Date of publication:
**20.04.2005 Bulletin 2005/16**

(51) Int Cl.⁷: **G01K 11/00**

(21) Application number: **04256324.7**

(22) Date of filing: **14.10.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **16.10.2003 KR 2003072255**

(71) Applicants:
• **Samsung Electronics Co., Ltd.**
**Suwon-si, Gyeonggi-do (KR)**
• **Nizhny Novgorod Microwave Research Institute, Ltd.**
**Nizhny Novgorod 603089 (RU)**

(72) Inventors:
• **Lee, Jeonghwan**
**Suwon-si Gyeonggi-do (KR)**
• **Rassadovsky, Vyacheslav A.**
**Nizhny Novgorod (RU)**

• **Belov, I.F.**
**Nizhny Novgorod (RU)**
• **Tereschenkov, V.I.**
**Nizhny Novgorod (RU)**
• **Aleshina, T. N.**
**Nizhny Novgorod (RU)**
• **Obolensky, V. A.**
**Nizhny Novgorod (RU)**
• **Sobolev, Oleg**
**Nizhny Novgorod (RU)**
• **Kleshnin, Sergey N.**
**Nizhny Novgorod (RU)**

(74) Representative: **Greene, Simon Kenneth**
**Elkington and Fife LLP,**
**Prospect House,**
**8 Pembroke Road**
**Sevenoaks, Kent TN13 1XR (GB)**

(54) **Method and radio-thermometer for measuring electromagnetic energy radiated from inside of human body**

(57)    A radio-thermometer system and method for measuring temperature of the inside of the human body are provided. The radio-thermometer system includes: an antenna, which receives thermal energy radiated from an object whose temperature needs to be measured; first and second noise sources, which are maintained at different temperatures in order to obtain a reflection coefficient Γ of an interface between the antenna and the object; a first switch, which periodically switches an output of the first or second noise source in response to a first pulse signal; a circulator, which adds a signal received by the antenna to a reflective wave output from the first or second noise source via the first switch and transmits an addition result in one direction; third and fourth noise sources, which are maintained at different temperatures; a second switch, which periodically switches an output of the third noise source, an output of the circulator, or an output of the fourth noise source in response to a second pulse signal, the second pulse signal being in synchronization with the first pulse signal; and an amplifier, which amplifies an output of the second switch. It is possible to achieve high precision and reproducibility of measurement of electromagnetic waves emitted from the human body by solving the problem of impedance mismatch at an interface between an antenna and the human body.

FIG. 3

**Description**

The present invention relates to a radio-thermometer system, and more particularly, to a radio-thermometer system and method for measuring electromagnetic energy radiated from the inside of the human body.

Ordinary objects emit electromagnetic energy in a predetermined frequency band at an absolute temperature of 0° K or higher. On the other hand, some objects completely absorb energy around them and emit electromagnetic energy in nearly all frequency bands, and these objects are called black bodies according to Plank's radiation law.

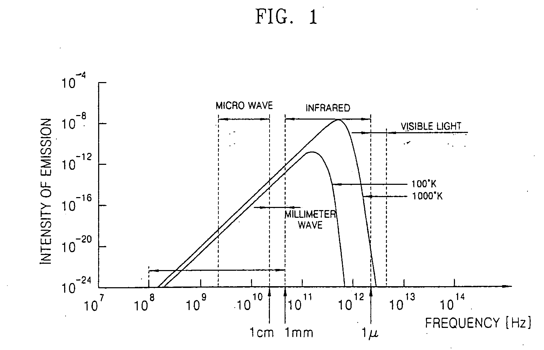

FIG 1 illustrates the variation of the intensity of radiant energy of a black body. Referring to FIG. 1, the intensity of radiant energy of a black body varies from frequency to frequency and reaches its peak in an infrared ray range of 3 - 15 μm. Generally, radiant energy of a black body in the infrared ray range can be detected using an infrared ray camera, and radiant energy of the black body in a microwave range can be detected using a radio-thermometer having a directional antenna and a high sensitivity receiver. Radio-thermometers, which were first adopted in the astronomical field, have been mostly used for measuring energy radiated from planets or stars in the universe and estimating the temperatures of the planets or stars.

Given that in an infrared ray range, the human skin has almost the same energy characteristics as black bodies, a distribution of temperatures on the surface of the human body can be obtained by measuring energy radiating from the human skin in the infrared ray range. Recently, radio-thermometers have been increasingly used for receiving energy from tissues in the human body and measuring temperatures of the tissues.

The human skin, however, does not serve as a black body in a microwave frequency range such that some of the electromagnetic energy radiated from inner tissues deep inside the human body is transmitted to the human skin. The intensity of the electromagnetic energy transmitted from the inner tissues of the human body to the human skin may vary depending on the type of a medium, through which the electromagnetic energy has reached the human skin, for example, depending on whether the electromagnetic energy has reached the human skin through muscles, through bones, or through fat. Radio-thermometers using microwaves estimate the temperature of the inside of the human body by measuring electromagnetic energy radiated from the human body having a frequency of 1 GHz - 6 GHz at the skin of the human body.

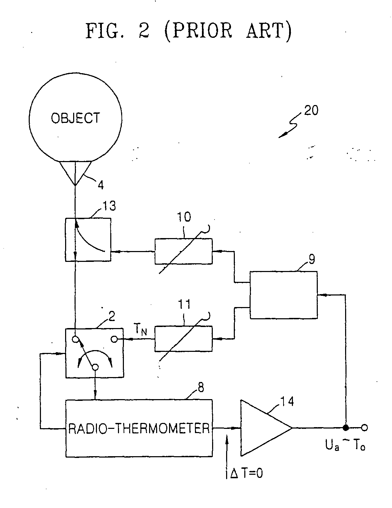

FIG. 2 is a diagram illustrating a conventional radio-thermometer system 20 using microwaves. Referring to FIG. 2, a signal output from a noise source 9, which is controllable, is input to a first attenuator 10, which is an adjustable, and then to a directional coupler 13, which is connected to an object 1 whose temperature needs to be measured via an antenna 4. The signal output from the controllable noise source 9 is also input to a second attenuator 11, which is adjustable, and then to one terminal of a switch 2. The other terminal of the switch 2 receives energy emitted from the object 1 and energy reflected from the object 1. The switch 2 is periodically switched by a clock pulse generator in a radio-thermometer 8. A signal output from the radio-thermometer 8 is provided to an output terminal via an integrator 14 as a voltage Ua corresponding to temperature To of the object 1. The conventional radio-thermometer system 20 measures the temperature of the inside of the object 1 by adjusting the noise source 9 several times.

The conventional radio-thermometer system 20, however, may cause many problems during measuring the temperature of the inside of the object 1 due to impedance mismatch at an interface between the antenna 4 and the object 1 or interference of electromagnetic waves in and around the conventional radio-thermometer system 20. The problem of the interference of electromagnetic waves can be solved by using an electromagnetic wave shielded room. However, the problem of impedance mismatch at the interface between the antenna 4 and the object 1 is responsible for lack of reproducibility and thus causes errors.

Therefore, it is necessary to develop a new radio-thermometer which can precisely measure the temperature of the object 1 even though impedance mismatch occurs at the interface between the antenna 4 and the object 1.

According to an aspect of the present invention, there is provided a radio-thermometer system comprising: an antenna, which receives thermal energy radiated from an object whose temperature needs to be measured; first and second noise sources, which are maintained at different temperatures in order to obtain a reflection coefficient $\Gamma$ of an interface between the antenna and the object; a first switch, which periodically switches an output of the first or second noise source in response to a first pulse signal; a circulator, which adds a signal received by the antenna to a reflective wave output from the first or second noise source via the first switch and transmits an addition result in one direction; third and fourth noise sources, which are maintained at different temperatures; a second switch, which periodically switches an output of the third noise source, an output of the circulator, or an output of the fourth noise source in response to a second pulse signal, the second pulse signal being in synchronization with the first pulse signal; and an amplifier, which amplifies an output of the second switch.

The temperature T of the object may be obtained by using the following equation:

$$T_A = \alpha[T(1- \Gamma) + T_{rec}\Gamma]$$

where $\alpha$ denotes a signal transmission coefficient of the antenna, and $T_{rec}$ denotes an effective noise temperature of the radio-thermometer system. The reflection coefficient $\Gamma$ may be obtained by the following equation:

$$\Gamma = \frac{\Delta U \cdot M}{\alpha \cdot 2\Delta T}$$

where $\Delta U = \Delta U''^k_{st2} - \Delta U'^k_{st2} = \Delta U''^k_{st1} - \Delta U'^k_{st1}$,

$$M = \frac{\alpha(1 - \Gamma_{st})(T^k_{st2} - T^k_{st1})}{(\Delta U^k_{st2} - \Delta U^k_{st1})},$$

$T^k_{st1}$ and $T^k_{st2}$ are the first and second reference temperatures, respectively, which are used in calibrating the radio-thermometer system, $\Delta U'^k_{st1}$ denotes an output of the radio-thermometer system when the radio-thermometer system contacts an object having the first reference temperature $T^k_{st1}$, and the first noise source is connected to the radio-thermometer system, $\Delta U''^k_{st1}$ denotes an output of the radio-thermometer system when the radio-thermometer system contacts the object having the first reference temperature $T^k_{st1}$, and the second noise source is connected to the radio-thermometer system, $\Delta U'^k_{st2}$ denotes an output of the radio-thermometer system when the radio-thermometer system contacts an object having the second reference temperature $T^k_{st2}$, and the first noise source is connected to the radio-thermometer system, and $\Delta U''^k_{st2}$ denotes an output of the radio-thermometer system when the radio-thermometer system contacts the object having a first reference temperature $T^k_{st2}$, and the second noise source is connected to the radio-thermometer system.

According to another aspect of the present invention, there is provided a method of measuring electromagnetic energy radiated from the human body by using a radio-thermometer system, comprising the operations of: connecting an antenna to an object whose temperature needs to be measured; providing first through fourth noise sources; enabling a first switch to periodically switch an output of the first source or an output of the second source in response to a first pulse signal; adding a signal received by the antenna to the output of the first or second noise source and transmitting an addition result in one direction; enabling a second switch to periodically switch an output of the third noise source, the addition result, or an output of the fourth noise source in response to a second pulse signal, the second pulse signal being in synchronization with the first pulse signal; and amplifying an output of the second switch to a predetermined voltage level.

The method of measuring electromagnetic energy radiated from the human body by using a radio-thermometer system may further include the operation of calibrating the radio-thermometer system by using objects having the first and second reference temperatures.

The present invention provides a radio-thermometer, which estimates the temperature of the inside of the human body based on the temperature of an antenna measured by automatically calculating a reflection coefficient of an interface between the antenna and the human body and compensating for impedance mismatch between them by as much as the reflection coefficient.

The present invention also provides a method of measuring electromagnetic waves radiated from the human body.

According to the present invention, it is possible to achieve high precision and reproducibility of measurement of electromagnetic waves emitted from the human body by solving the problem of impedance mismatch at an interface between an antenna and the human body.

The above and other features and advantages of the present invention will become more apparent by describing in detail exemplary embodiments thereof with reference to the attached drawings in which:

FIG. 1 is a graph illustrating Planck's radiation law;
FIG. 2 is a diagram illustrating a conventional radio-thermometer system using microwaves;
FIG. 3 is a diagram illustrating a radio-thermometer system according to an exemplary embodiment of the present invention;

FIG. 4 is a graph illustrating the waveforms of outputs of first and second switches of the radio-thermometer system of FIG. 3; and

FIG. 5 is a graph illustrating the waveform of an output from the radio-thermometer system of FIG. 3.

The present invention will now be described more fully with reference to the accompanying drawings, in which exemplary embodiments of the invention are shown. In the drawings, like reference numerals represent like elements.

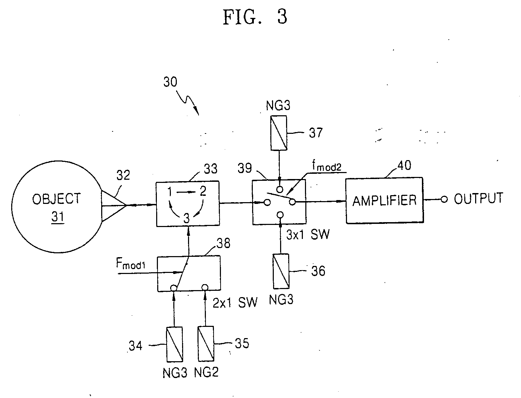

FIG. 3 is a diagram illustrating a radio-thermometer system 30 according to an exemplary embodiment of the present invention. Referring to FIG. 3, the radio-thermometer system 30 includes an antenna 32, which receives radiant energy radiated from an object 31, a circulator 33, first through fourth noise sources 34 through 37, first and second switches 38 and 39, and an amplifier 40. Each of the first through fourth noise sources 34 through 37 is set to a reference temperature, which is higher or lower than the temperature of energy radiated from the object 31. In the present embodiment, the object 31 is the human body. Thus, the first and third noise sources 34 and 35, which provide a reference temperature for the temperature of the object 31, are set to a first reference temperature, i.e., 32 °C, which is lower than the normal temperature of the human body, i.e., 36.5 °C, and the second and fourth noise sources 35 and 37, which also provide a reference temperature for the temperature of the object 31, are set to a second reference temperature, i.e., 42°C, which is higher than 36.5 °C.

The circulator 33 adds a signal received by the antenna 32 to a reflective wave output from the first or second noise source 34 or 35 and transmits the addition result to the second switch 39. The first switch 38 is periodically switched to transmit the first noise source 34 or the second noise source 35 to the circulator 33 in response to a first pulse signal Fmod1. The second switch 39 is periodically switched to transmit the third noise source 36, the circulator 33, or the fourth noise source 37 to the amplifier 40 in response to a second pulse signal Fmod2. The first and second pulse signals Fmod1 and Fmod2 are provided by a typical digital pulse divider circuit and are in synchronization with each other. The amplifier 40 amplifies a signal output from the second switch 39 to a predetermined level because the magnitude of the signal is very small.

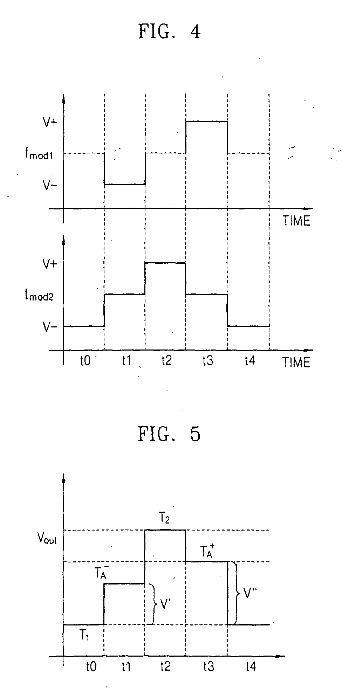

FIG. 4 illustrates the waveforms of outputs of the first and second switches 38 and 39. Referring to FIG. 4, during a period of time from 0 to t0, the first switch 38 has no output level, and the second switch 39 has an output level of V-, which is equal to an output level of the third noise source 36. During a period of time from t0 to t1, the first switch 38 has an output level of V-, which is equal to an output level of the first noise source 34, and the second switch 39 has the same output level as the circulator 33. During a period of time from t1 to t2, the first switch 38 has no output level, and the second switch 39 has an output level of V+, which is equal to an output level of the fourth noise source 37. During a period of time from t2 to t3, the first switch 38 has the same output level as the second noise source 35, and the second switch 39 has the same output level as the circulator 33. During a period of time from t3 to t4, the first switch 38 has no output level, and the second switch 39 has an output level of V-, which is equal to the output level of the third noise source 36. The waveforms of the signals output from the first and second switches 38 or 39 during a period of time from 0 to t3 are repeated afterwards.

As shown in FIG. 4, the second switch 39 has the same output level as the circulator 33 between 0 to t1 and between t3 to t4, which means the signal output from the first or second noise source 34 or 35 is well transmitted to the circulator 33 and then added to the signal received by the antenna 32, and the addition result is well transmitted to the second switch 39.

A total of four signal sources are provided in the radio-thermometer system 30, i.e., first through fourth signal sources. The first signal source is a result $T_A^-$ of adding a signal $T_A$ received by the antenna 32 to the output of the first noise source 34. The second signal source is a result $T_A^+$ of adding the signal $T_A$ received by the antenna 32 to the output $T_1$ of the second noise source 35. The third signal source is the output of the third noise source 36. The fourth signal source is the output $T_2$ of the fourth noise source 37. The waveform of an output of the radio-thermometer system 30 in response to the output of the first or second noise source 34 or 35 is illustrated in FIG. 5.

An output $U_i$ of the radio-thermometer system 30 in response to a predetermined signal source can be defined by Equation (1) below:

$$U_i = kT_i + u_0 \qquad\qquad (1)$$

where $k$ is a transmission coefficient, $T_i$ is an effective noise temperature of the predetermined signal source, and $u_0$ is a constant, which is independent from the predetermined signal source.

Outputs $U_1$, $U_2$, and $U_A$ of the radio-thermometer system 30 in response to the third signal source $T_1$, the

fourth signal source $T_2$, and the signal received by the antenna 32 $T_A$, respectively, can be obtained by using Equations (2) below:

$$U_1 = kT_1 + u_0 \qquad (2)$$

$$U_2 = kT_2 + u_0$$

$$U_A = kT_A + u_0$$

Equations (2) are rearranged into Equation (3) below:

$$T_A = T_1 + M\Delta U_A \qquad (3)$$

where $M = \dfrac{\Delta T_k}{\Delta U_k}$, $\Delta T_k = T_2 - T_1$, and $\Delta U_k = U_2 - U_1$.

The signal $T_A$ received by the antenna 32, i.e., temperature at the antenna 32, can be expressed by Equation (4) below:

$$T_A = \alpha[T(1-\Gamma)+T_{rec}\Gamma] \qquad (4)$$

where $\alpha$ denotes a signal transmission coefficient of the antenna 32, T denotes the temperature of the object 31 to be measured, $T_{rec}$ denotes an effective noise temperature of a reception terminal of the radio-thermometer system 30, and $\Gamma$ denotes a reflection coefficient.

Equation (4) is taught by K.M. Ludeke, et al. in "A New Radiation Balance Microwave Thermograph for Simultaneous and Independent Noise Temperature and Emissivity Measurements" (Journal of Microwave Power and Electromagnetic Energy, Vol. 14, No. 2, 1979).

Equation (4) can be expanded into Equations (5) or (6) by calibrating the radio-thermometer system 30 using objects having the first and second reference temperatures.

$$\alpha[T_{st1}^{k}(1 - \Gamma_{st}) + (T_0 - \Delta T)\Gamma_{st}] = T_1 + M\Delta U_{st1}^{k}$$

$$\alpha[T_{st1}^{k}(1 - \Gamma_{st}) + (T_0 - \Delta T)\Gamma_{st}] = T_1 + M\Delta U''^{k}_{st1} \qquad (5)$$

$$\alpha[T_{st2}^{k}(1 - \Gamma_{st}) + (T_0 - \Delta T)\Gamma_{st}] = T_1 + M\Delta U_{st2}^{k}$$

$$\alpha[T_{st2}^{k}(1 - \Gamma_{st}) + (T_0 - \Delta T)\Gamma_{st}] = T_1 + M\Delta U''^{k}_{st2} \qquad (6)$$

where $T_{st1}^{k}1$ denotes the first reference temperature, i.e., 32 °C, and $T_{st2}^{k}$ denotes the second reference temperature, i.e., 42 °C.

The effective noise temperature $T_{rec}$ of the reception terminal can be expressed by Equation (7) below:

$$T_{rec} = T_0 \pm \Delta T \qquad (7)$$

where To is an effective noise temperature of a port of the antenna 32, which is equal to the temperature of an HF module maintained by a temperature adjustment apparatus in the HF module, and $\Delta T$ denotes a difference in the temperatures of the third and fourth noise sources 36 and 37, which amounts to 5 °C that is half of 10°C, i.e., the difference between the temperatures of the third and fourth noise sources 36 and 37. Here, the HF module is attached to a rear portion of the antenna 32.

A pair of equations in Equations (6) are added to each other, and a pair of equations in Equations (7) are added to each other, thereby obtaining Equations (8) below:

$$\alpha[T^k_{st1}(1-\Gamma st)+T_0\Gamma_{st}]=T_1+M\Delta\bar{U}^k_{st1}$$

$$\alpha[T^k_{st2}(1-\Gamma_{st})+T_0\Gamma_{st}]=T_1+M\Delta\bar{U}^k_{st2} \qquad (8)$$

where $\Delta\bar{U}^k_{st1}$ denotes an average temperature of the radio-thermometer system 30 when the first noise source 34 is connected to the radio-thermometer system 30, and $\Delta U^k_{st2}$ denote an average temperature of the radio-thermometer system 30 when the second noise source 35 is connected to the radio-thermometer system 30.

Equations (8) can be rearranged into Equation (9) below:

$$M = \frac{\alpha(1-\Gamma_{st})(T^k_{st2}-T^k_{st1})}{(\Delta U^k_{st2}-\Delta U^k_{st1})} \qquad (9)$$

Equation (10) below can be obtained from Equations (6) and (7):

$$\Gamma = \frac{\Delta U \cdot M}{\alpha \cdot 2\Delta T} \qquad (10)$$

where $\Delta U = \Delta U^{"k}_{st2} - \Delta U^{'k}_{st2} = \Delta U^{"k}_{st1} - \Delta U^{'k}_{st1}$. Here, $\Delta U^{'k}_{st1}$ denotes an output of the radio-thermometer system 30 when the radio-thermometer system 30 contacts an object having the first reference temperature $T^k_{st1}$, i.e., 32 °C, and the first noise source 34 is connected to the radio-thermometer system 30, $\Delta U^{"k}_{st1}$ denotes an output of the radio-thermometer system 30 when the radio-thermometer system 30 contacts the object having the first reference temperature $T^k_{st1}$, i.e., 32 °C, and the second noise source 35 is connected to the radio-thermometer system 30, $\Delta U^{'k}_{st2}$ denotes an output of the radio-thermometer system 30 when the radio-thermometer system 30 contacts an object having the second reference temperature $T^k_{st2}$, i.e., 42 °C and the first noise source 34 is connected to the radio-thermometer system 30, and $\Delta U^{"k}_{st2}$ denotes an output of the radio-thermometer system 30 when the radio-thermometer system 30 contacts the object having a first reference temperature $T^k_{st2}$, i.e., 42 °C, and the second noise source 35 is connected to the radio-thermometer system 30.

As shown in Equation (10), the reflection coefficient $\Gamma$ is proportional to a difference between the output of the radio-thermometer system 30 when the first noise source 34 is connected to the radio-thermometer system 30 and the output of the radio-thermometer system 30 when the second noise source 35 is connected to the radio-thermometer system 30.

Therefore, the temperature $T$ of the object 31 can be obtained by substituting the temperature $T_A$ at the antenna 32 and the reflection coefficient $\Gamma$ into Equation (4). Accordingly, the radio-thermometer system 30 can solve the problem of impedance mismatch between the antenna 32 and the object 31.

The radio-thermometer system 30 according to the present invention was applied to various phantoms each having a constant temperature, and the results are shown in Table (1) below.

Table (1)

| Reference Temperature of Phantom (°C) | Temperature of Phantom Measured by Radio-thermometer (°C) | Standard Deviation (sigma) |
|---|---|---|
| 32.00 | 31.98 | 0.06 |
| 34.00 | 33.94 | 0.05 |

Table (1)   (continued)

| Reference Temperature of Phantom (°C) | Temperature of Phantom Measured by Radio-thermometer (°C) | Standard Deviation (sigma) |
|---|---|---|
| 35.00 | 34.95 | 0.14 |
| 36.00 | 36.00 | 0.04 |
| 37.00 | 37.00 | 0.04 |
| 38.00 | 37.99 | 0.04 |
| 40.00 | 39.97 | 0.04 |
| 42.00 | 41.97 | 0.04 |

More specifically, Table (1) shows results of measuring temperatures of phantoms twenty times by using the radio-thermometer system 30, averaging the measured temperatures of each of the phantoms, and calculating a standard deviation of the measured temperatures of each of the phantoms. The measured temperatures of each of the phantoms except for those of the phantom having a temperature of 35°C have a very small standard deviation and are different from one another by less than 0.1 °C. Therefore, the radio-thermometer system 30 can achieve high precision and reproducibility of measurement of the temperature of the object 31.

While the present invention has been particularly shown and described with reference to exemplary embodiments thereof, it will be understood by those of ordinary skill in the art that various changes in form and details may be made therein without departing from the scope of the present invention as defined by the following claims.

**Claims**

1.   A radio-thermometer system comprising:

an antenna, which receives thermal energy radiated from an object whose temperature needs to be measured;
first and second noise sources, which are maintained at different temperatures in order to obtain a reflection coefficient $\Gamma$ of an interface between the antenna and the object;
a first switch, which periodically switches an output of the first or second noise source in response to a first pulse signal;
a circulator, which adds a signal received by the antenna to a reflective wave output from the first or second noise source via the first switch and transmits an addition result in one direction;
third and fourth noise sources, which are maintained at different temperatures;
a second switch, which periodically switches an output of the third noise source, an output of the circulator, or an output of the fourth noise source in response to a second pulse signal, the second pulse signal being in synchronization with the first pulse signal; and
an amplifier, which amplifies an output of the second switch.

2.   The radio-thermometer system of claim 1, wherein the temperature T of the object is obtained by using the following equation:

$$T_A = \alpha[T(1 - \Gamma) + T_{rec}\Gamma]$$

where $\alpha$ denotes a signal transmission coefficient of the antenna, and $T_{rec}$ denotes an effective noise temperature of the radio-thermometer system,
the reflection coefficient $\Gamma$ is obtained by the following equation:

$$\Gamma = \frac{\Delta U \cdot M}{\alpha \cdot 2\Delta T}$$

where $\Delta U = \Delta U''^k_{st2} - \Delta U''^k_{st2} = \Delta U''^k_{st1} - \Delta U'^k_{st1}$ ,

$$M = \frac{\alpha(1-\Gamma_{st})(T^k_{st2}-T^k_{st1})}{(\Delta U^k_{st2}-\Delta U^k_{st1}},$$

$T^k_{st1}$ and $T^k_{st2}$ are the first and second reference temperatures, respectively, which are used in calibrating the radio-thermometer system, $\Delta U'^k_{st1}$ denotes an output of the radio-thermometer system when the radio-thermometer system contacts an object having the first reference temperature $T^k_{st1}$, and the first noise source is connected to the radio-thermometer system, $\Delta U''^k_{st1}$ denotes an output of the radio-thermometer system when the radio-thermometer system contacts the object having the first reference temperature $T^k_{st1}$, and the second noise source is connected to the radio-thermometer system, $\Delta U'^k_{st2}$ denotes an output of the radio-thermometer system when the radio-thermometer system contacts an object having the second reference temperature $T^k_{st2}$, and the first noise source is connected to the radio-thermometer system, and $\Delta U''^k_{st2}$ denotes an output of the radio-thermometer system when the radio-thermometer system contacts the object having a first reference temperature $T^k_{st2}$, and the second noise source is connected to the radio-thermometer system.

3. The radio-thermometer system of claim 2, wherein the first reference temperature is set to 32°C, and the second reference temperature is set to 42°C.

4. The radio-thermometer system of claim 1, 2 or 3, wherein the first and third noise sources are maintained at the same temperature, and the second and fourth noise sources are maintained at the same temperature.

5. The radio-thermometer system of claim 4, wherein the first and third noise sources are maintained at a temperature of 32°C, and the second and fourth noise sources are maintained at a temperature of 42°C.

6. A method of measuring electromagnetic energy radiated from the human body by using a radio-thermometer system, comprising the operations of:

   connecting an antenna to an object whose temperature needs to be measured;
   providing first through fourth noise sources;
   enabling a first switch to periodically switch an output of the first source or an output of the second source in response to a first pulse signal;
   adding a signal received by the antenna to the output of the first or second noise source and transmitting an addition result in one direction;
   enabling a second switch to periodically switch an output of the third noise source, the addition result, or an output of the fourth noise source in response to a second pulse signal, the second pulse signal being in synchronization with the first pulse signal; and
   amplifying an output of the second switch to a predetermined voltage level.

7. The method of claim 6, wherein the first and third noise sources are maintained at the same temperature, and the second and fourth noise sources are maintained at the same temperature.

8. The method of claim 6 or 7, wherein the first and third noise sources are maintained at a temperature of 32°C, and the second and fourth noise sources are maintained at a temperature of 42°C.

9. The method of claim 6, 7 or 8 further comprising the operation of calibrating the radio-thermometer system by using objects having the first and second reference temperatures.

10. The method of claim 9 having a reflection coefficient, which is proportional to a difference between an output of the radio-thermometer system when the radio-thermometer system contacts an object having the first reference temperature and the first noise source is connected to the radio-thermometer system and an output of the radio-thermometer system when the radio-thermometer system contacts the object having the first reference temperature and the second noise source is connected to the radio-thermometer system.

11. The method of claim 9 having a reflection coefficient, which is proportional to a difference between an output of the radio-thermometer system when the radio-thermometer system contacts an object having the second reference temperature and the first noise source is connected to the radio-thermometer system and an output of the radio-thermometer system when the radio-thermometer system contacts the object having the second reference temperature and the second noise source is connected to the radio-thermometer system.

FIG. 1

## FIG. 2 (PRIOR ART)

OBJECT

20

RADIO-THERMOMETER

$T_N$

$\Delta T = 0$

$U_a \sim T_o$

FIG. 3

FIG. 4

FIG. 5

**European Patent Office**

**EUROPEAN SEARCH REPORT**

Application Number

EP 04 25 6324

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | FR 2 650 390 A (INST NAT SANTE RECH MED) 1 February 1991 (1991-02-01) * page 6, line 26 - page 13, line 20 * * page 15, paragraph 2 * ----- | 1,2,6,9 | G01K11/00 |
| X | WO 01/67057 A (LAND DAVID VICTOR ; UNIV GLASGOW (GB)) 13 September 2001 (2001-09-13) * page 12, line 28 - page 17, line 11; figures 4-6 * ----- | 1-3,6,8,9 | |
| X | US 5 949 845 A (STERZER FRED) 7 September 1999 (1999-09-07) * the whole document * ----- | 1,3,6,8,9 | |
| A | US 4 235 107 A (KOHLER JURGEN ET AL) 25 November 1980 (1980-11-25) * the whole document * ----- | 1,6 | |
| | | | TECHNICAL FIELDS SEARCHED (Int.Cl.7) |
| | | | G01K |

The present search report has been drawn up for all claims

| Place of search | Date of completion of the search | Examiner |
|---|---|---|
| The Hague | 10 December 2004 | Ramboer, P |

EPO FORM 1503 03.82 (P04C01)

**ANNEX TO THE EUROPEAN SEARCH REPORT
ON EUROPEAN PATENT APPLICATION NO.**

EP 04 25 6324

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2004

| Patent document cited in search report | | Publication date | Patent family member(s) | | Publication date |
|---|---|---|---|---|---|
| FR 2650390 | A | 01-02-1991 | FR | 2650390 A1 | 01-02-1991 |
| | | | US | 5176146 A | 05-01-1993 |
| WO 0167057 | A | 13-09-2001 | AU | 3762401 A | 17-09-2001 |
| | | | CA | 2402943 A1 | 13-09-2001 |
| | | | EP | 1264164 A1 | 11-12-2002 |
| | | | WO | 0167057 A1 | 13-09-2001 |
| | | | JP | 2003526103 T | 02-09-2003 |
| | | | US | 2003026321 A1 | 06-02-2003 |
| US 5949845 | A | 07-09-1999 | US | 5688050 A | 18-11-1997 |
| US 4235107 | A | 25-11-1980 | DE | 2803480 A1 | 02-08-1979 |
| | | | FR | 2415799 A1 | 24-08-1979 |
| | | | GB | 2013350 A ,B | 08-08-1979 |
| | | | JP | 1326214 C | 16-07-1986 |
| | | | JP | 54113381 A | 04-09-1979 |
| | | | JP | 60046648 B | 17-10-1985 |

For more details about this annex : see Official Journal of the European Patent Office, No. 12/82